# EUROPEAN PATENT SPECIFICATION

(11) **EP 0 646 008 B1**
(45) Date of publication and mention of the grant of the patent: **15.12.1999**
(21) Application number: 93913781.6
(22) Date of filing: 03.05.1993
(51) Int. Cl.: A61K 31/56, A61K 31/565, A61K 31/58, A61K 31/57

(54) **MINIMIZING PROGESTIN ASSOCIATED BREAKTHROUGH BLEEDING**
MINIMIERUNG VON MIT PROGESTERON VERBUNDENEN DURCHBRUCKBLUTUNGEN
PROCEDE POUR REDUIRE AU MINIMUM LES HEMORRAGIES UTERINES SECONDAIRES LIEES A LA PRISE DE PROGESTERONE

(30) Priority: 06.05.1992 WO PCT/US92/03574
(43) Date of publication of application: 05.04.1995
(73) Proprietor: MEDICAL COLLEGE OF HAMPTON ROADS, Norfolk, VA 23507 (US)
(72) Inventor: HODGEN, Gary D., Norfolk, VA 23507 (US)
(74) Representative: Hermans, Franciscus G.M.
(86) International application number: US9304003
(87) International publication number: WO9321927

(56) References cited:
- WO-A-94/04156
- FERTIL.STERIL., vol. 53, no. 4, 1990 pages 747-50, 'Intereference with ovulation by sequential treatment with the antiptrogesterone RU486 and synthetic progestin'
- FERTIL.STERIL., vol. 60, no. 4, October 1993 pages 610-15, 'Sequential regimen of the antiprogesterone RU486 and synthetic progestin for contraception'
- CANCER RES., vol. 47, no. 14, 1987 pages 3367-71, 'Prolactin release-inhibitory effects of progesterone, megestrol acetate, and mifepristone (RU 38486) by cultured rat pituitary tumour cells'
- CHEMICAL ABSTRACTS, Volume 109, No. 15, CONCANNON et al., "Effects of the Antiprogestin RU 486 on Progesterone-Dependent Uterine Development and Bioassay of Progestational Activity in Estrogen-Primed Immature Female Dogs", Abstract No. 122618x, Acta Endocrinol, (Copenhagen), 1988; 118(3), pp. 389-398.
- Drug Facts and Comparisons, 1989, Oral Contraceptives, Progestin-Only Products, p. 108f.
- CHEMICAL ABSTRACTS, Volume 115, No. 11, BATISTA et al., "Daily Administration of the Progesterone Antagonist RU 486 Prevents Implantation in the Cycling Guinea Pig", Abstract No. 106339a, Am. J. Obstet. Gynecol., 1991, 165(1), p. 82-86.
- Drug Facts and Comparisons, 1991, Levonorgestrel Implant Contraceptive System, p. 108g-108j.

## Description

### FIELD OF THE INVENTION

The invention relates to the use of anti-progestin for the manufacture of a medicament for minimizing breakthrough bleeding in users of progestin-only pharmaceutic preparations, such as contraceptives.

### BACKGROUND OF THE INVENTION

The primate menstrual cycle is characterized by a proliferation and regression of the uterine lining under the control of steroid hormones, primarily estrogen and progesterone. It is believed that the staggered cyclic levels of hormones contribute to the growth and shedding of the upper tissue compartment of the uterus.

The endometrium on the uterus is characterized by distinct layers, such as the stratum functionalis and stratum basalis. It is the functionalis which represents the transient upper tissue compartment that is shed during menstruation.

It is believed that the basalis serves as a source of new cells for the regeneration of the functionalis in succeeding cycles. Wilborn & Flowers, Seminars in Reproductive Endocrinology 2:4, 307, 1984; Padykula et al., Biology of Reproduction 40, 681, 1989. If the basalis does serve as a germinal layer, then the effects of damage to the basalis during a given cycle could be manifest in succeeding cycles.

Because endometrial proliferation serves to prepare the uterus for an impending pregnancy, manipulation of hormones and of the uterine environment can serve as suitable targets for contraception. For example, estrogens are known to decrease follicle stimulating hormone secretion by feedback inhibition.

Under certain circumstances, estrogens can also inhibit luteinizing hormone secretion, once again by negative feedback, although under normal circumstances it is believed that the spike of circulating estrogen found just prior to ovulation induces the surge of gonadotrophin hormones that occurs just prior to and resulting in ovulation. High doses of estrogen also can prevent conception probably due to interference with implantation.

Progesterone is responsible for the progestational changes of the endometrium and the cyclic changes of cells and tissues in the cervix and the vagina. For example, progesterone makes the cervical mucus thick, tenacious and cellular. It is believed that thickened mucus impedes spermatozoal transport.

Progesterone has somewhat of an anti-estrogenic effect on the myometrial cells, for example, decreasing the excitability of the smooth muscle cells, and the like. It is known that large doses of progesterone inhibit luteinizing hormone secretion and progesterone injections can prevent ovulation in humans.

The most prevalent form of oral contraception is a pill that combines both an estrogen and a progestagen, the so-called combined oral contraceptive preparations. Apparently, the estrogen and progestagen act in concert to block gonadotrophin release.

Alternatively, there are oral contraceptive preparations that comprise a progestagen only. Such preparations are indicated particularly for individuals who have experienced side effects or an intolerance to the combined preparations or in lactating women because of the lack of an estrogenic effect on lactation.

However, the progestagen-only preparations have a more varied spectrum of side effects than do the combined preparations. A disadvantage of the progestagen-only preparations is the relatively high incidence of bleeding problems, such as, prevalent or heavier menstrual spotting, amenorrhea and more breakthrough bleeding. Thus, the combined preparations are the preferred oral contraceptives in use today. Sheth et al., Contraception 25, 243, 1982.

Some of the very common side effects of the progestagen-only oral contraceptives is the increased incidence of menstrual spotting, break through bleeding, variations in menstrual cycle length and occasionally amenorrhea.

Nevertheless, it would be preferable to ha an contraceptive preparation that minimizes the amounts of estrogens and progestagens used. For example, estrogens are known to cause dizziness, nausea, headache and breast tenderness. Thus, a progestagen-only contraceptive would forego such possible problems and be an improvement over the combined preparations if the above-referred to problems of progestagen-only contraceptives also can be remedied. George Washington University Medical Center, Population Reports, Series A, No. 3, September 1975.

Anti-progestins include inhibitors of progesterone synthesis, ligands, such as antibodies, to progesterone and progesterone receptor antagonists. For example, mifepristone (RU486) is a progesterone receptor antagonist. RU486 binds to the progesterone receptor and produces antagonistic effects. Following oral administration, RU486 in the human has a half life of about 20-24 hours. When administered in the luteal phase of the menstrual cycle, RU486 induces luteolysis and vaginal bleeding.

RU486 may act directly on the endometrium to induce vaginal bleeding. RU486-mediated luteolysis appears to be secondary to changes in gonadotrophin secretion and thus the effects are similar to those following exogenous progesterone administration. Baulieu, Science 245, 1351, 1989.

Swahn et al. (Human Reproduction 5(4), 402, 1990) relates to administering RU486 early during the luteal phase prior to implantation. Those authors found that a single dose of RU486 administered on the second day after the LH peak causes a retardation of endometrial development without upsetting the menstrual cycle. Those authors speculated that it may be possible that the effect on the endometrium may be sufficient to prevent implantation.

### SUMMARY OF THE INVENTION

It is an object of the instant invention to provide a method and means of enhancing the value progestin-only pharmaceutical preparations, such as contraceptives.

It is another object of the instant invention to provide a kit and/or program to enhance the every day use of progestin-only pharmaceutical preparations, such as contraceptives.

Those and other objects have been achieved with the use of a biologically effective amount of an anti-progestin for the manufacture of a medicament. for minimizing uterine bleeding in a female using a progestin-only pharmaceutical preparation.

The invention also relates to the use of biologically effective amounts of a progestin and an anti-progestin for the manufacture of a medicament for achieving birth control.

The invention also relates to an implant intended for subcutaneous or local administration comprising a pharmaceutically acceptable inert core material which would function as a matrix, a progestin and an anti-progestin.

The invention further relates to a kit of the "progestin-only" type comprising means for the daily administration of a progestin in a contraceptively effective amount, characterised in that the kit also provides means for the administration of an anti-progestin.

### BRIEF DESCRIPTION OF THE DRAWINGS

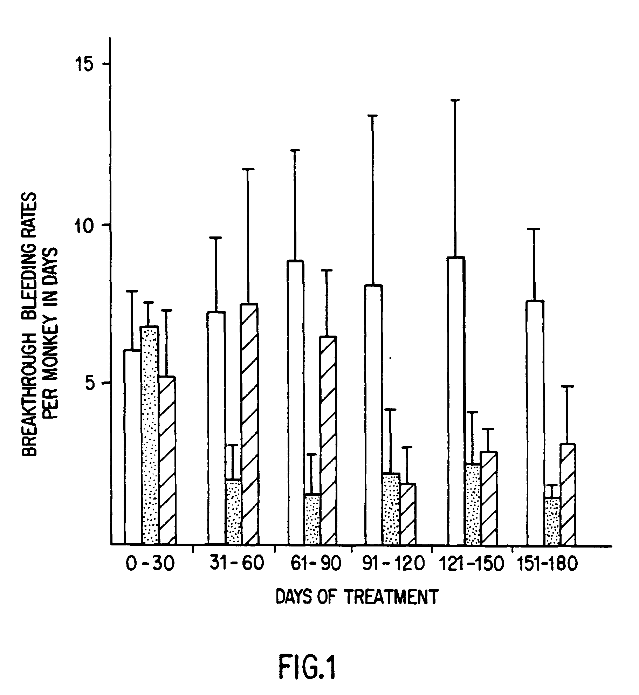

Figure 1 depicts the breakthrough bleeding rate per monkey over the course of treatment. Individual monkeys were given a progestin-only contraceptive and intermittent dosing of anti-progestin. Breakthrough bleeding was scored on all days except for the seven days following an RU486 dose. The average duration of bleeding following an RU486 dose was 3.2 ± 1.1 days. The incidence of RU486-induced menses was 100% within 72 hours. Open bars indicate monkeys receiving oral contraceptive, levonorgestrel, only. Stippled bars represent monkeys receiving levonorgestrel and RU486 at days 30, 60, 90, 120, 150 and 180. Cross-hatched bars indicate monkeys receiving levonorgestrel and RU486 at days 90 and 180. The daily dose of levonorgestrel was 10 µg per day orally except on the day when RU486 was given when no progestin was administered. RU486 was administered orally and intermittently at 50 mg per dose.

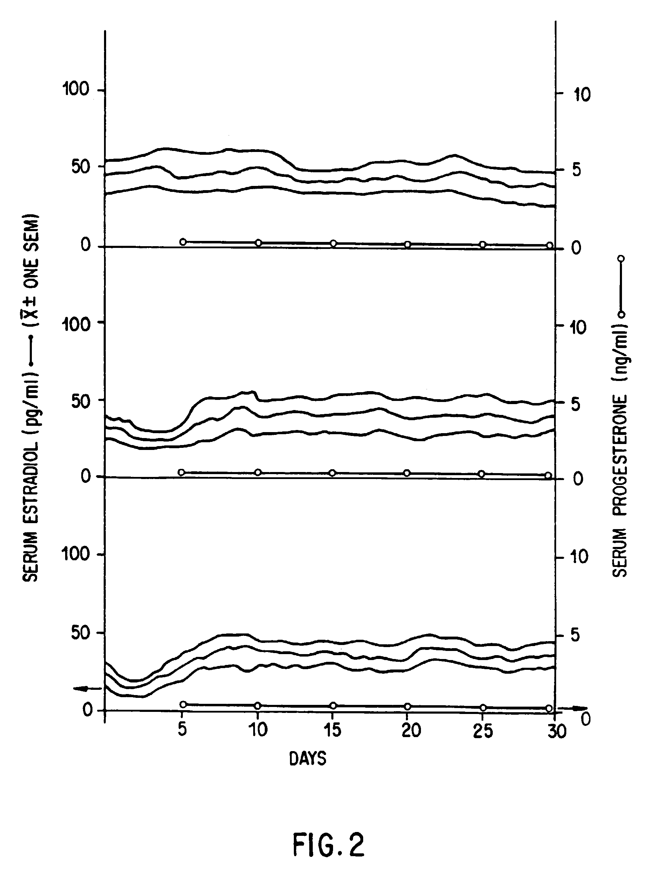

Figure 2 depicts serum estradiol and progesterone levels in monkeys of the various treatment groups. The lower limit of detection of estradiol was 12 pg/ml. The lower limit of detection of progesterone was 0.2 ng/ml. The top panel depicts animals receiving progestin only on a daily basis. The middle panel depicts animals receiving progestin daily and RU486 on days 30, 60, 90 and 120. The bottom panel depicts animals receiving progestin daily and RU486 on day 90 only. The progestin was levonorgestrel.

### DETAILED DESCRIPTION OF THE INVENTION

The instant invention relates to means for reducing irregular bleeding in those users of progestagen-only pharmaceutical preparations, such as contraceptives. The invention relates to the use of an anti-progestin in combination with the progestagen-only pharmaceutical preparations, such as a contraceptive. For the purposes of the instant invention, progestin and progestagen are considered synonyms.

Progestagen-only pharmaceutical preparations, such as tablets which can be administered orally, vaginal rings, implant systems (biodegradable or not), injectables and transdermal systems, which can be used as contraceptives are known in the art.

For example, commonly used oral contraceptives contain the synthetic progestins, cingestol, ethynodiol diacetate, lynestrenol, norethindrone, norgestrel, quingestanol acetate, levonorgestrel (active ingredient of NORPLANT), norethisterone, chlormadinone, megestrol, desogestrel, gestodene, norgestimate and the like. Fotherby, Journal of Drug Development 4 (2), 101, 1991. Essentially any progestin suitable for use in a progestagen-only pharmaceutical can be used in the practice of the instant invention.

The anti-progestin can be an inhibitor of progesterone synthesis, such as epostane, azastene or trilostane (Creange, Contraception 24, 289, 1981; Drugs of the Future 7, 661, 1982; van der Spuy at al., Clin. Endo. 19, 521, 1983; Birgerson at al., Contraception 35, 111, 1987; U.S. Pat. No. 3296255) or a progesterone receptor antagonist, or any such pharmaceutically suitable agent that counteracts the normal biological activity of progesterone, such as antibodies or ligands bindable to progestins or to the progesterone receptor.

A suitable anti-progestin is a progesterone receptor antagonist. For example, RU486, Onapristone, Org 31710 ((6α,11β,17β)-11-(4-dimethylaminophenyl)-6-methyl-4',5'-dihyrospiro [estra-4,9-diene-17,2'(3'H)-furan]-3-one), Org 33628 ((11β,17α)-11-(4-acetylphenyl)-17, 23-epoxy-19, 24-dinorchola-4,9,20-trien-3-one) and Org 31806 ((7β,11β,17β)-11-(4-dimethylaminophenyl)-7-methyl-4',5'-dihydrospiro(estra-4,9-diene-17,2' (3'H)-furan]-3-one) are particularly suitable in the practice of the instant invention. U.S. Pat. No. 4386085.

The anti-progestin can be administered by way of any art-recognized means practiced in the pharmaceutic arts. For example, a suitable anti-progestin may be so formulated so that it can be administered orally, via a skin patch for transdermal absorption, contained within an inert matrix which is implanted within the body and in the implanted state is released slowly, such an implant is taught in U.S. Patent Has. 4957119 and 5088505 and the like.

Thus, pharmaceutic formulations of solid dosage forms include tablets, capsules, cachets, pellets, pills, powders or granules; topical dosage forms include solutions, powders, fluid emulsions, fluid suspensions, semi-solids, ointments, pastes, creams, gels or jellies and foams; and parenteral dosage forms includes solutions, suspensions, emulsions or a dry powder comprising an effective amount of anti-progestin as taught in the instant invention.

It is known in the art that the active ingredient, the anti-progestin, can be contained in such formulations in addition to pharmaceutically acceptable diluents, fillers, disintegrates, binders, lubricants, surfactants, hydrophobic vehicles, water soluble vehicles, emulsifiers, buffers, humectants, moisturizers, solubilizers, preservatives and the like. The means for administration are known in the art and an artisan can refer to various pharmacologic references for guidance, see, for example, "Modern Pharmaceutics", Banker & Rhodes, Marcel Dekker, Inc. 1979; "Goodman & Gilman's The Pharmaceutical Basis of Therapeutics", 6th Edition, MacMillan Publishing Co., New York 1980.

In the case of oral contraceptives, it is known that the kits thereof contain a pill for each day of the month (either 28 days, the lunar month, or 30 days) wherein any one pill may contain one or more of the active ingredients.

The effective amount of an anti-progestin in the practice of the instant invention can be determined using art-recognized methods, for example, by establishing dose-response curves in suitable animal models and extrapolating therefrom to humans, extrapolating from suitable in vitro systems or by determining effectiveness in clinical trials. The determination of an effective dose is a routine exercise in the pharmaceutic arts. The artisan will take into account various physical parameters of the prospective host such as weight, age and the like.

In like vein, the dosage regimen of the preparation is determinable using art-recognized methods such as establishing a dose response curve in similar primate models or in a suitable in vitro experimental system or by an empirical determination in clinical trials.

It is contemplated, in view of the dynamic state of the endocrine system in primates, that administration of the anti-progestin can be either on a tonic or continuous basis, such as in parallel with administration of a progestin-only oral contraceptive, or on an episodic basis because of the dynamic relationship of the endometrial cells and the long term effects of anti-progestins. Thus, the anti-progestin can be administered in combination with the progestin in the form of a pill or as a co-component in an implant or the progestin can be given in one form and the anti-progestin can be given in another form, for example, the progestin may be given in the form of a pill and the anti-progestin can be delivered as a component of an implant. Alternatively, the progestin can be administered daily whereas the anti-progestin is to be administered monthly, or at other intermittent intervals.

In the case of the progesterone receptor antagonists RU486, Org 33628, Org 31806 and Org 31710, it is anticipated that a suitable human oral dose will be on the order of 10-250 mg per dose. The amount per dose can be lowered or raised based on the number of doses actually given, that is the interval at which the doses of anti-progestin are administered and characteristics of the individual receiving the treatment and the potency of a particular anti-progestin.

The number of doses can vary from monthly to longer intervals taking into consideration cost, safety and the like. Thus, a suitable regimen is having the anti-progestin administered every thirty days, every sixty days or every ninety days. Alternatively, in the case of contraceptives where many of the pill kits are configured based on the lunar month, the anti-progestin can be administered on the twenty-eighth day of each cycle. Variations of dosage based on route of administration may vary and such changes can be determined practicing known techniques as described above.

The present invention is described further below with respect to specific examples which are tended to illustrate the instant invention without limiting the scope thereof.

### EXAMPLE

In the present study, laboratory primates (Macaca fasicularis, n = 18), having normal ovulatory menstrual cycles, were assigned at random to one of three groups: Group I (n = 6) received 10 µg of levonorgestrel daily by oral ingestion for 180 days. Group II (n = 6) was given the same dose regimen of levonorgestrel as in Group I, except that 50 mg of RU486 was administered orally and intermittently on treatment days 30, 60, 90, 120, 150 and 180. Similarly, Group III primates (n = 6) received levonorgestrel daily, but RU486 on treatment days 90 and 180. For Groups II and III, levonorgestrel was withheld only on the days that the anti-progestin was given.

Breakthrough bleeding was recorded daily based on presence or absence of blood in the vagina upon insertion of a saline-moistened cotton-tipped applicator. Menstrual bleeding that occurred within 7 days after each RU486 treatment was not counted as breakthrough bleeding.

To determine whether the dose of levonorgestrel reliably blocked ovulation, all primates were bled daily from the femoral vein (3.0 ml) from treatment day 91 to 120, so that serum estradiol and progesterone levels could be determined by radioimmunoassay using known materials and techniques.

Intermittent RU486 treatment in animals receiving a progestin daily markedly reduced irregular menstrual bleeding by 69% on average, whether the interval between treatments of the anti-progestin was 30 or 90 days (p < 0.05). However, there was a trend (p > 0.05) toward rising breakthrough bleeding in the 2nd and especially 3rd month after RU486 treatment (Group III).

That the daily dose regimen of levonorgestrel effectively blocked ovulation was evident from the absence of overt serum progesterone elevations. The intermittent doses of anti-progestin did suppress transiently mean tonic serum estradiol to below 30 pg/ml for four or five days; otherwise ovarian estrogen secretion was non-episodic (48 ± 11 pg/ml, Group I; 41 ± 6 pg/ml, Group II; and 42 ± 9 pg/ml; Group III).

More importantly, intermittent administration of RU486 significantly reduced irregular menstrual bleeding whether the every 30 day or every 90 day anti-progestin regimen was employed, albeit the anti-progestin impact appeared to fade with less frequent dosing.

The bleeding control effect of RU486 was manifest for two to three months. The anti-progestin may have imparted certain long-lasting functional characteristics in basal endometrial cells. The primate data show the effectiveness of combining progestin with an anti-progestin, without the need for exogenous estrogen, to control endometrial bleeding.

It should be noted that the efficacy of the progestin to block ovulation is not compromised by the intermittent administration of an anti-progestin. See Figure 2.

While the invention has been described in detail and with reference to certain embodiments thereof, it would be apparent to one skilled in the art that various changes and modifications can be made.

## Claims

1. The use of a biologically effective amount of an anti-progestin for the manufacture of a medicament for minimizing uterine bleeding in a female using a progestin-only pharmaceutical preparation.

2. A use according to claim 1, characterised in that the anti-progestin is a compound that inhibits progesterone synthesis or is a progesterone receptor antagonist.

3. A use according to claim 2, characterised in that the antagonist is RU 486, Org 33628, Org 31806, or Org 31710.

4. A use according to claim 1, characterised in that the progestin is desogestrel.

5. A use according to claim 1, characterised in that the medicament is in the form of a pill or tablet.

6. A use according to claim 1, characterised in that the medicament is in the form of an implant.

7. A use according to claim 3, characterised in that the medicament contains the antagonist in an amount of 10 mg to 250 mg per dose.

8. A use according to claim 3, characterised in that the medicament is to be administered once every 30 days.

9. A use according to claim 3, characterised in that the medicament is to be administered once every 60 days.

10. A use according to claim 3, characterised in that the medicament is to be administered once every 90 days.

11. A use according to claim 3, characterised in that the medicament is to be administered on the 28th day of a cycle.

12. The use of a composition comprising biologically effective amounts of a progestin and an antiprogestin for the manufacture of a medicament for achieving birth control by the uninterrupted administration of the progestin.

13. A use according to claim 12, characterised in that the progestin is desogestrel

14. A use according to claim 12 or 13, characterised in that the anti-progestin is RU 486, Org 33628, Org 31806, or Org 31710.

15. A use according to claim 14, characterised in that the progestin is desogestrel and the antiprogestin is Org 33628.

16. An implant intended for subcutaneous or local administration comprising a pharmaceutically acceptable core material which would function as a matrix, a progestin, and an anti-progestin.

17. A contraceptive kit of the "progestin-only" type comprising means for the daily administration of a progestin in a contraceptively effective amount, characterised in that the kit also provides means for the administration of an anti-progestin

18. A contraceptive kit according to claim 17, characterised in that the kit provides means for the administration of the anti-progestin once every 30 days.

## Patentansprüche

1. Verwendung einer biologisch wirksamen Menge eines anti-Progestins zur Herstellung eines Medikaments, das Uterusblutungen einer Frau auf ein Minimum zurückführt, unter Verwendung eines nur Progestin enthaltenden pharmazeutischen Präparats.

2. Verwendung gemäss Anspruch 1, dadurch gekennzeichnet, dass das anti-Progestin eine Verbindung ist, die die Progesteron-Synthese inhibiert oder die ein Antagonist des Progesteron-Rezeptors ist.

3. Verwendung gemäss Anspruch 2, dadurch gekennzeichnet, dass der Antagonist RU486, Org 33628, Org 31806 oder Org 31710 ist.

4. Verwendung gemäss Anspruch 1, dadurch gekennzeichnet, dass das Progestin Desogestrel ist.

5. Verwendung gemäss Anspruch 1, dadurch gekennzeichnet, dass das Medikament in Form einer Pille oder Tablette ist.

6. Verwendung gemäss Anspruch 1, dadurch gekennzeichnet, dass das Medikament in Form eines Implantats ist.

7. Verwendung gemäss Anspruch 3, dadurch gekennzeichnet, dass das Medikament den Antagonisten in einer Menge von 10 mg bis 250 mg pro Dosis enthält.

8. Verwendung gemäss Anspruch 3, dadurch gekennzeichnet, dass das Medikament einmal alle 30 Tage verabreicht wird.

9. Verwendung gemäss Anspruch 3, dadurch gekennzeichnet, dass das Medikament einmal alle 60 Tage verabreicht wird.

10. Verwendung gemäss Anspruch 3, dadurch gekennzeichnet, dass das Medikament einmal alle 90 Tage verabreicht wird.

11. Verwendung gemäss Anspruch 3, dadurch gekennzeichnet, dass das Medikament am 28. Tag eines Zyklus verabreicht wird.

12. Verwendung einer Zusammensetzung, die biologisch wirksame Mengen eines Progestins und eines anti-Progestins zur Herstellung eines Medikaments zur Geburtenkontrolle durch die ununterbrochene Gabe von Progestin umfasst.

13. Verwendung gemäss Anspruch 12, dadurch gekennzeichnet, dass das Progestin Desogestrel ist.

14. Verwendung gemäss Anspruch 12 oder 13, dadurch gekennzeichnet, dass das anti-Progestin RU 486, Org 33628, Org 31806 oder Org 31710 ist.

15. Verwendung gemäss Anspruch 14, dadurch gekennzeichnet, dass das Progestin Desogestrel und das anti-Progestin Org 33628 ist.

16. Implantat zur subkutanen oder lokalen Anwendung, welches ein pharmazeutisch annehmbares Kernmaterial, das als eine Matrix fungiert, sowie ein Progestin und ein anti-Progestin umfasst.

17. Empfängnisverhütendes Set des Typs der nur Progestin enthält, welches Mittel zur täglichen Verabreichung eines Progestins in einer für eine Empfängnisverhütung wirksamen Menge umfasst, dadurch gekennzeichnet, dass das Set auch Mittel zur Verabreichung eines anti-Progestins umfasst.

18. Empfängnisverhütendes Set gemäss Anspruch 17, dadurch gekennzeichnet, dass das Set einmal alle 30 Tage Mittel zur Verabreichung des anti-Progestins zur Verfügung stellt.

## Revendications

1. Utilisation d'une quantité efficace du point de vue biologique d'une anti-progestine pour la fabrication d'un médicament destiné à réduire à un minimum un saignement utérin chez une femme utilisant une préparation pharmaceutique à base de progestine uniquement..

2. Utilisation suivant la revendication 1, caractérisée en ce que l'anti-progestine est un composé qui inhibe la synthèse de la progestérone ou est un antagoniste de récepteur de progestérone.

3. Utilisation suivant la revendication 2, caractérisée en ce que l'antagoniste est RU 486. Org 33628, Org 31806 ou Org 31710.

4. Utilisation suivant la revendication 1, caractérisée en ce que la progestine est le désogestrel.

5. Utilisation suivant la revendication 1, caractérisée en ce que le médicament est sous la forme d'une pilule ou d'un comprimé.

6. Utilisation suivant la revendication 1, caractérisée en ce que le médicament est sous la forme d'un implant.

7. Utilisation suivant la revendication 3, caractérisée en ce que le médicament contient l'antagoniste en une quantité de 10 mg à 250 mg par dose.

8. Utilisation suivant la revendication 3, caractérisée en ce que le médicament doit être administré une fois tous les 30 jours.

9. Utilisation suivant la revendication 3, caractérisée en ce que le médicament doit être administré une fois tous les 60 jours.

10. Utilisation suivant la revendication 3, caractérisée en ce que le médicament doit être administré une fois tous les 90 jours.

11. Utilisation suivant la revendication 3, caractérisée en ce que le médicament doit être administré le 28ème jour d'un cycle.

12. Utilisation d'une composition comprenant des quantités efficaces du point de vue biologique d'une progestine et d'une anti-progestine pour la fabrication d'un médicament destiné à permettre un contrôle des naissances par administration ininterrompue de la progestine.

13. Utilisation suivant la revendication 12, caractérisée en ce que la progestine est le désogestrel.

14. Utilisation suivant les revendications 12 ou 13, caractérisée en ce que l'anti-progestine est RU 486. Org 33628, Org 31806 ou Org 31710.

15. Utilisation suivant la revendication 14, caractérisée en ce que la progestine est le désogestrel et l'anti-progestine est Org 33628.

16. Implant conçu pour l'administration sous-cutanée ou locale comprenant un matériau de noyau acceptable du point de vue pharmaceutique qui va fonctionner en tant que matrice, une progestine et une anti-progestine.

17. Kit contraceptif du type, à base de progestine uniquement, comprenant un moyen pour l'administration quotidienne d'une progestine en une quantité efficace du point de vue contraceptif, caractérisé en ce que le kit fournit aussi un moyen pour l'administration d'une anti-progestine.

18. Kit contraceptif suivant la revendication 17, caractérisé en ce que le kit fournit un moyen pour l'administration de l'anti-progestine une fois tous les 30 jours.
